Europäisches Patentamt

European Patent Office

Office européen des brevets

(19)

(11) Publication number : **0 484 266 A2**

# (12) EUROPEAN PATENT APPLICATION

(21) Application number : **91500119.2**

(22) Date of filing : **28.10.91**

(51) Int. Cl.$^5$: **A23L 1/30,** A23D 9/00,
     A23J 7/00

(30) Priority : **30.10.90 ES 9002759**

(43) Date of publication of application :
     **06.05.92 Bulletin 92/19**

(84) Designated Contracting States :
     **AT BE CH DE DK ES FR GB GR IT LI LU NL SE**

(71) Applicant : **UNION INDUSTRIAL Y
     AGRO-GANADERA S.A.- UNIASA
     Camino de Purchil, 66
     E-18004 Granada (ES)**

(72) Inventor : **Gil, Angel
     Parque del Genil, Edificio Esmeralda
     E-18004 Granada (ES)**
     Inventor : **Moreno, José
     Pueblo Mediterráneo D-9
     E-18190 Cenes de la Vega, Granada (ES)**
     Inventor : **Jiménez, Jes s
     Primavera, 4
     E-18320 Granada (ES)**

(74) Representative : **Ibanez, José Francisco
     Rodriguez San Pedro, 10
     E-28015 Madrid (ES)**

(54) **Fat mixture for infant and adult nutrition.**

(57)   Fat mixture for infant and adult nutrition, basically comprising a mixture of phospholipids from the cerebrum of domestic animals, at least one vegetable oil, and/or at least an animal fat, and/or at least a fish oil, and/or medium chain triglycerides, in which the ratios between oleic/linoleic/alpha-linolenic fatty acids, between arachidonic/docosahexaenoic fatty acids, and its phospholipids content are similar to those of human milk and mediterranean diet.

EP 0 484 266 A2

The invention is related to an edible fat mixture which can be used in an isolated form or combined with other nutritional products. Said products can be infant formulas or dietetic products for nutrition of adults, both healthy and ill.

At present several families of polyunsaturated fatty acids, in short PUFA, are known to exist. The n6 series, which is considered essential to human life, derived from linoleic acid (18:2n6). The n3 series, which is now considered essential during early postnatal life in the human beings, derived from alpha-linolenic acid (18:3n3). The n9 series derived from oleic acid (18:1n9) and the n7 series from palmitoleic acid (16:1n7). These two families can be synthesized endogenously.

Long chain PUFA (more than 18 carbon atoms) are synthesized from their precursors, via succesive desaturation and elongation. In each of said families exist fatty acids with similar chain length and unsaturation degree, however, none of the members of one family is exactly the same as the corresponding members of the other family, since the families are unique, metabolically derived from different precursors, can not be interconverted and have different functions.

In addition, a competition is known to exist between alpha-linolenic (18:3n3), linoleic (18:2n6) and oleic (18:1n9) acids for the enzyme 6-desaturase, which presents a strong affinity for alpha-linolenic acid. Studies have demonstrated that the ratio linoleic/alpha-linolenic influences the membrane composition of the metabolites of the n6 and n3 series. Also, that a high value of linoleic/alpha-linolenic ratio in the diet can supress the elongation and desaturation of alpha-linolenic acid to its long chain metabolites and increases the unfavourable accumulation of metabolites of the n6 series in body tissues. It has also been demonstrated that 5-desaturation is inhibited by an excess of linoleic and alpha-linolenic acids, and the 6-desaturation by the presence of the superior homologues of the series n6 and n3. In addition, the eicosapentaenoic (20:5n3) and docosahexaenoic (22:6n3) acids are competitive inhibitors of cyclo-oxygenase and lipo-oxygenase for the arachidonic acid (20:4n6), which affects the production of prostaglandins, thromboxanes and leucotrienes of the series 2.

The principal lipids found in animal membranes are cholesterol and phospholipids, the ratio between both components being a determining factor on the fluidity and functionality of the membrane. The contribution of the phospholipids to the membrane fluidity will depend on the balance between the different phospholipids present and the nature of the acyl groups.

The dietary balance between fatty acids of the n6 and n3 series is a significant factor for the composition of fatty acids in the membranes. In addition, the intake of oleic acid partially affects the conversion of the linoleic and alpha-linolenic acids in long chain PUFA and, therefore, the composition of membrane phospholipids.

The accumulation of docosahexaenoic acid (22:6n3) in membranes, particularly in the gray matter of the cerebrum and in the retina, can be detrimentally lowered by an increase in the ratio linoleic/alpha-linolenic in the diet. For example, safflower and sunflower oils, and other fats with a high ratio 18:2n6/18:3n3, increase in the retina and cerebrum the levels of 22:5n6 created from 18:2n6 (linoleic), and limit the content of 22:6n3 (docosahexaenoic), which is synthesized from 18:3n3 (alpha-linolenic). In addition, a diet based on linseed or soy oils, which possesses a low ratio 18:2n6/18:3n3, increases the content of 20:5n3 (eicosapentaenoic) in the phospholipids, but not the content of 22:6n3 (docosahexaenoic). This suggests the existence of a low level of 4-desaturase activity in humans.

Artificial formulas on the market today, both for adult and infant nutrition, are usually characterised in their composition of fatty acids, by relatively lows levels of oleic acid and high levels of linoleic acid. Most of them do no contain alpha-linolenic acid and also do not contain long chain PUFA, both of the n6 and n3 series, particularly 20:4n6 (arachidonic) and 22:6n3 (docosahexaenoic).

For example, patent GB-2067587B discloses a fat mixture for infant nutrition products which is composed of 10-15% of lauric oil (coconut, babasu, palm kernel), 20-50% of palm oil, 10-25% of oleic oils (olive, oleo), and 0-20% of linoleic oil (corn, soy, sunflower, safflower).

The main problems connected with this example of fat mixture for infant nutrition products are:

a) Low content of alpha-linolenic acid (18:3n3), in fact its presence is not specified. As a result, there is an inadequate ratio between oleic, linoleic and alpha-linolenic acids.

b) High content of lauric acid (12:0), which increases susceptibility towards atherogenesis.

c) High level of palmitic acid (16:0), which increases the probability of clot formation, thus decreasing the absorption of fat.

d) Absence of long chain PUFA.

Also, European patent 0129990 discloses a fat mixture for infant nutrition products having a composition of 30% of palm oil, 20% of oleic oil (olive and oleinate), 27% of lauric oil, 22% of linoleic oil (soy) and 1% of lecithin. The advantages of an adequate ratio between oleic, linoleic and alpha-linolenic acids is not mentioned, and high levels of lauric (12.7%), linoleic (16-19%) and alpha-linolenic (2.4%) acids are proposed. The disclosed fat mixture does not content long chain PUFA.

In relation with the sources of long chain PUFA, two biological sources have been used till now:

1) Fish: Generally blue fish, because its higher content of fat. The fatty acid composition of fish oil is characterised by low levels of saturated fatty acids and long chain PUFA of the n6 series. In the other hand, fish oil presents high levels of long chain PUFA of the n3 series, particularly 20:5n3 (eicosapentaenoic) and 22:6n3 (docosahexaenoic). However, the exclusive utilization of fish oil as a source of PUFA states several problems resulting from the lack of balance in the ratio between PUFA of n3 and n6 series. The ratio between long chain PUFA of n3 and n6 series is very high in this source, which leads to a very low value in the ratio between arachidonic and docosahexaenoic (20:4n6/22:6n3), which is not recommended in a general diet. In addition, most types of fish have a high content of 20:5n3 (eicosapentaenoic) and a low content of 22:6n3 (docosahexaenoic). This alters the ratio 20:5n3/22:6n3 which, for example, in human milk is about 1/2-3 and in fish oil ranges between 2-3/1.

2) Egg: Its chemical composition is characterised by fat levels about 10%, most of which comes from the yolk. Within the fatty acids composition of egg, is remarkable the high level of saturated fatty acids, about 36-40%, the absence of alpha-linolenic acid (18:3n3) and the reduced presence of long chain PUFA of the n6 and n3 series. The major problem in using egg as a source of long chain PUFA, is its high content in cholesterol, 500 mg/100 g, which brings it unsuitable for the preparation of adult diets. In addition, the low content of long chain PUFA in this source makes it necessary to use a high percentage of egg fat in the total fat of the artificial formula, thus the cost becoming very high.

Moreover, the effects on the percentage of the plasmatic content of long chain PUFA in children fed during 21 days with an infant formula containing PUFA derived from eggs (Diet I), with an infant formula without PUFA (Diet II) and with human milk (Diet III) have been described. The plasmatic percentages of PUFA obtained are those appearing in the following diagram, with particular reference to arachidonic and docosahexaenoic acids.

|          | Arachidonic (20:4n6) | Docosahexaenoic (22:6n3) |
|----------|----------------------|--------------------------|
| Diet III | 1.20                 | 0.64                     |
| Diet I   | 0.90                 | 0.41                     |
| Diet II  | 0.49                 | 0.26                     |

In the diagram it can be observed that, although Diet I raises the plasmatic percentages, the value is still far lower from that in human milk, at least for these two fatty acids.

U.S. patent 4,670,285 describes a fat mixture for infant nutrition based on the lipids of egg yolk (75-95%), also containing coconut and soy oils, and contemplating the possible addition of fish oil. The use of this fat mixture creates some problems. The cholesterol content is very high, making the mixture unsuitable for adult nutrition products. In addition, there is a low ratio of long chain PUFA in egg lipids, which are the carriers of these acids in the mixture. This implies a high supply of egg lipids to the mixture in order to reach a fair amount of long chain PUFA. There is a low content, in the fat mixture described, of alpha-linolenic acid (0.3-0.4%). The mixture with tilapia oil does not provide docosahexaenoic acid (22:6n3), and there is a high ratio 20:5n3/20:4n6.

German patent 3603000 describes a fat mixture with long chain PUFA, obtained by mixing animal and/or vegetable fats, preferably liver fat, egg lipids, oleo, corn oil, soybean oil, palm oil, palm kernel oil, or coconut oil and fish oil. It does not disclose an optimal ratio between 18:1n9 (oleic), 18:2n6 (linoleic) and 18:3n3 (alpha-linolenic) acids. The cholesterol content of this mixture is very high, and it is not appropriate for adult nutrition, particularly, cardiovascular patients. In addition, a ratio 2.5/1 for 20:4n6 (arachidonic) and 22:6n3 (docosahexaenoic) acids is very high when compared with the ranges in human milk, of 0.1-1/0.1-1. The incorporation of phospholipids to the mixture is not considered.

French patent 2553261 describes an artificial milk containing phospholipids from animal placenta, as source of PUFA. In this fat mixture, there are high levels of strogens which could alter the metabolism of human beings. Comparing its composition of fatty acids to human milk, there is a high level of palmitic acid (16:0) and a low content of oleic acid (18:1n9). As far as the PUFA content, there is no alpha-linolenic acid, and the level of arachidonic acid is very high, which alters the ratio 20:4n6/22:6n3, 5/1 in the mixture, compared to the range of values in human milk, 0.1-1/0.1-1.

In view of these antecedents and taking into account the role in the diet of the 18:1n9 (oleic), 18:2n6 (linoleic) and 18:3n3 (alpha-linolenic) acids and their derivatives as regulators of the formation of long chain PUFA and on the metabolism of eicosanoids, it would be desirable to know the suitable intake levels for optimal condition of the membranes. Moreover, since no direct data are available about these levels, it would be desirable to obtain this information from other factors, as the composition of human milk and from the dietary intake of fatty acids in populations with low risk of cardiovascular diseases, such as the mediterranean countries.

Although oleic acid is not essential for humans, it plays an important role in the maintenance of the membrane structure, and its intake causes changes in the absorption of fats and in cholesterol metabolism. Infants fed with artificial formulas in which linoleic acid is predominant, present plasmatic cholesterol levels considerably lower (110 mg/dl) than those fed with artificial formulas in which oleic acid is predominant (133 mg/dl). Infants fed with artificial formulas in which oleic acid is predominant, displayed higher HDL-cholesterol levels and also of apoproteins A-I and A-II than those which received formulas in which the linoleic acid is predominant. The cholesterol ratio of LDL-VLDL/HDL was found to be lower in children who received the formula in which the oleic acid was predominant.

Currently, monounsaturated fatty acids, in short MUFA, (oleic and palmitoleic), are receiving considerable attention in relation with the prevention of atherosclerosis and cardiovascular diseases. It has been shown that a diet rich in olive oil and with low levels of saturated fats co-exist with a low incidence of ischemic diseases. Also, it has been shown that young humans fed with a diet rich in MUFA display lower levels of LDL and HDL compared to those fed with a control diet or a diet enriched in PUFA.

It has also been demostrated that a diet enriched with olive oil increases HDL-cholesterol levels in elderly people, while a diet enriched with PUFA lowers these levels, both in elderly people and in people with normal or high levels of triglycerides.

Therefore, a desirable fat mixture would be that which contained a ratio of fatty acids, MUFA and PUFA, such that the reducing effects on the level of plasmatic cholesterol would be parallel with the increase of the levels of HDL.

A deficiency of linoleic acid has been detected in mammals and in humans who receive fat-free parenteral nutrition or in children fed with skim milk. The human requeriments for linoleic acid, have been estimated to be 1% of the total caloric intake.

Recent studies suggest that linoleic acid alone may not be sufficient to completely satisfy the requeriments for essential fatty acids during the fetal period and the early postnatal period. A requeriment for alpha-linolenic acid and/or long chain PUFA could be established on the basis that an alteration in the distribution of fatty acids affects visual and mental functions in humans fed with a fat free parenteral solution.

Recently, it has been demonstrated that in experimental animals the levels of prostaciclin PGI2, which displays anticlotting properties, are increased when the diet is rich in olive oil, while decreased when the diet is enriched with corn oil. At the same time, lower levels of thromboxane TXB2 are found in animals fed with diets enriched in olive oil compared to animals fed with a diet enriched with PUFA.

Having into account that fatty acids as 20:3n6 (eicosatrienoic), 20:4n6 (arachidonic) and 20:5n3 (eicosapentaenoic) are the precursors of three different prostaglandin series and other related eicosanoids, which have specific properties and regulate different physiological functions, the alteration of the proportion of these fatty acids in the diet may cause changes in the composition of tissue membranes and in the individual's physiology.

On the other hand, an adequate supply of these fatty acids and of 22:6n3, a particularly important compound in the development of the cerebrum and the retina, could be critical in human development. Again would be desirable to have orientating factors of the distribution of fatty acids in the diet, such as human milk and the so-called mediterranean diet. It has been demonstrated that pre and postnatal children which were malnourished exhibited a significant decrease in 22:6n3 (docosahexaenoic) acid levels in the retina, particularly in those who were fed with a diet in which the ratio between n6/n3 fatty acids was altered.

The supplementation with long chain PUFA of adult diets is especially important where some diseases are present, such as cirrhosis and Crohn's disease. For these diseases, it has been observed that despite a normal linoelic and alpha-linolenic acids intake in the diet and the existence of normal levels of these fatty acids in the plasma, there is an alteration in the profiles of long chain PUFA of n3 and n6 series, which suggests an alteration in the mechanisms of desaturation and/or elongation of the precursors. In these cases, it would be particularly important to incorporate preformed long chain PUFA into the diet.

In recent years an increasing number of studies have considered the the possible negative effects that could be derived from an excessive amount of PUFA in the diet. Although the long term effects have not been established, the short term effects seem to indicate an increase in the degree of unsaturation of membrane lipids, with a corresponding increase of the susceptibility of lipids to oxidation, and an increase in tocopherol requirements. Epidemiological and on animal studies suggest that an excess of PUFA intake could enhance the action of known carcinogens. A relationship has also been shown between and excessive intake of PUFA and breast cancer. It seems that the increase in the peroxidation of lipids observed with an excess of PUFA in the diet could be responsible for the high incidence of tumors.

Arachidonic acid (20:4n6) is the precursor of the series 2 of eicosanoids, and an excess of this metabolite of the n6 series, or an excess of its precursors, in the diet may lead to an increase in thrombogenesis, a decrease in bleeding time, an increase in the inflammatory response of polymorphonuclear monocytes and leukocytes, as well as an increase in smooth muscle reactivity to allergies. In contrast, a diet predominantly based on long chain PUFA of the n3 series, such as the diet of the Eskimos, produce an increase of bleeding time, and a low incidence of diseases as atherosclerosis, arthritis, asthma and cardiovascular diseases. This is due to the fact that these acids are the precursors for the series 3 of eicosanoids.

The present invention provides a new edible fat mixture which possesses adequate levels of phospholipids and adeuqte ratios between oleic, linoleic and alpha-linolenic acids, as well as adequate levels of long chain PUFA, both of the n6 and n3 series. In addition, fat mixtures according to the invention posses an adequate ratio between arachidonic and docosahexaenoic acids. These fat mixtures are modeled on the fatty acids content of human milk for infant diets or formulas, and on the mediterranean diet for adult nutrition products. These diets promote the growth and development of the infants and contribute to improve nutrition and health condition, as well as prevention and treatment of some diseases, in adults.

Human milk contains about 4g/dl of lipids, made up from the following components: about 98% are triglycerides, 0.8% phospholipids and 0.3% cholesterol. In relation with composition of fatty acids, human milk generally contains: oleic acid (18:1n9) ranges between 30-40%; palmitic (16:0), stearic (18:0) and myristic (14:0) acids respectively range about 20-25%, 5-7% and 4-7%. The linoleic acid content normally varies between 6-16% and alpha-linolenic acid between 1.2-1.3% of total fatty acids content.

The average composition of fatty acids in human milk (Spanish mothers) and the approximate percentage intervals of the fatty acids of primary interest for the invention, are shown in Table 1:

## Table 1

| Fatty acid | Mean content +/- MSE* | Intervals (%) approx. |
|---|---|---|
| 10:0 | 1.78 +/- 0.16 | |
| 12:0 | 7.15 +/- 0.36 | |
| 14:0 | 6.48 +/- 0.31 | |
| 15:0 | - - - - - | |
| 16:0 | 16.96 +/- 0.31 | |
| 16:1n7 | 3.21 +/- 0.12 | |
| 17:0 | - - - - - | |
| 18:0 | 4.80 +/- 0.16 | |
| 18:1n9 | 40.14 +/- 0.90 | 30 - 45 |
| 18:2n6 | 16.07 +/- 0.71 | 6 - 20 |
| 18:3n3 | 1.36 +/- 0.10 | 0.3 - 1.8 |
| 20:0 | - - - - - | |
| 20:2n6 | 0.50 +/- 0.71 | |
| 20:3n6 | 0.68 +/- 0.71 | |
| 20:4n6 | 0.66 +/- 0.71 | 0.1 - 1 |
| 20:5n3 | 0.21 +/- 0.10 | |
| 21:0 | - - - - - | |
| 22:4n6 | 0.10 +/- 0.10 | |
| 22:5n6 | - - - - - | |
| 22:5n3 | 0.25 +/- 0.10 | |
| 22:6n3 | 0.40 +/- 0.10 | 0.1 - 1 |

(* MSE = Means standard error)

Human milk contains both medium chain and long chain fatty acids, and is particularly rich in PUFA of the n6 and n3 series. All of these acids are especially abundant in the milk of mothers with premature children.

As indicated previously, the ratio oleic/linoleic/alpha-linolenic (18:1n9/18:2n6/18:3n3) is a regulating factor in the synthesis of long chain PUFA and eicosanoids. The value of this ratio in human milk ranges about 30-45/6-20/0.3-1.8.

Another important long chain PUFA ratio in the diet, is the ratio arachidonic/docosahexaenoic (20:4n6/22:6n3) due to the importance of 22:6n3 acid as a component of the membrane lipids in both the cerebrum and retina. In human milk this ratio ranges between 0.1-1/0.1-1 approximately.

With regards to adult nutrition, the diet of mediterranean countries is currently considered a desirable model due to the low incidence shown in these countries of diseases such as atherosclerosis, cardiovascular diseases and cancer.

The approximate fatty acids composition in the standard mediterranean diet and the approximate percentage intervals for fatty acids of primary interest for the invention are shown in Table 2:

### Table 2

| Fatty acid | Mean Content | Intervals (%) approx. |
|---|---|---|
| 14:0 | 0.6 | |
| 16:0 | 19.2 | |
| 16:1 | 0.4 | |
| 18:0 | 6.4 | |
| 18:1 | 58.1 | 30 − 80 |
| 18:2n6 | 12.0 | 3 − 18 |
| 18:3n3 | 0.9 | 0.3 − 3 |
| 20:4n6 | 0.1 | 0.1 − 2 |
| 20:5n3 | 0.6 | |
| 22:6n3 | 0.6 | 0.1 − 3 |

The ratio 18:1n9/18:2n6/18:3n3 (oleic/linoleic/alpha-linolenic) ranges about 30-80/3-18/0.3-3. For the long chain PUFA, the ratio 20:4n6/22:6n3 (arachidonic/docosahexaenoic) ranges about 0.1-2/0.1-3.

Having into account the low incidence of cardiovascular diseases observed in the countries having this diet, the above ratios of fatty acids could be taken as a reference model for preparing a diet for adult nutrition.

For diets destined to patients with specific pathologies, it would be desirable to supplement the diet with determined long chain PUFA. For example, for cardiovascular patients would be convenient to supplement the diet with long chain PUFA of the n3 series, specifically with eicosapentaenoic acid (20:5n3). For cirrhosis, would be advisable to supplement the diet with long chain PUFA of both the n3 and n6 series.

In addition, taking into account the importance of PUFA as structural components of membrane phospholipids, and in view that from these phospholipids, by the action of phospholipases, the long chain PUFA precursors of the eicosanoids are obtained, it would be desirable to associate the PUFA added to the diet with phospholipids. In human milk, phospholipids are present in a concentration ranging approximately between 23.8-81.5 mg/dl, equivalent to 0.7-0.8% of total lipids. Among phospholipids the most important fractions are: phosphatidyl choline 28-29%, phosphatidyl ethanolamine 26-27%, sphingomyelins 30-32%, phosphatidyl serine 5-6% and phosphatidyl inositol 4-5%.

The majority of long chain PUFA present in human milk are associated with phospholipids, and this reason reinforces the recommendation of associating with phospholipids the PUFA added to the diets.

In addition, it has been demonstrated that milk phospholipids can protect the gastric mucosa, as they mediate the cytoprotective effects of prostaglandins.

Table 3 shows the approximate fatty acids composition in phospholipids of the cerebrum of calf and pig, together with a comparison with the fatty acid composition of other sources of PUFA, for example egg yolks lipids used in US-4.670.285 and DE-3603000 patents, and placenta lipids of FR-2553261 patent.

## Table 3

| Fatty acid | Calf cerebrum | Pig cerebrum | Egg yolk (1) | Placenta (2) |
|---|---|---|---|---|
| 14:0 | 4.1 | -- | -- | 0.6 |
| 16:0 | 17.2 | 15.3 | 26.1 | 30.0 |
| 16:1n7 | 1.8 | 0.8 | 3.3 | 1.5 |
| 18:0 | 16.3 | 17.5 | 10.2 | 13.0 |
| 18:1n9 | 29.0 | 29.5 | 37.1 | 12.1 |
| 18:2n6 | 0.5 | 1.0 | 10.7 | 8.8 |
| 18:3n3 | 0.8 | 0.6 | 0.3 | -- |
| 18:3n6 | 0.1 | 0.3 | -- | -- |
| 20:2n6 | 0.6 | 0.6 | -- | 0.8 |
| 20:3n6 | 0.7 | 0.9 | -- | 4.0 |
| 20:4n6 | 8.9 | 11.4 | 1.5-2.8 | 16.0 |
| 20:5n3 | 0.5 | 0.3 | 0-0.1 | 0.1 |
| 22:4n6 | 5.1 | 4.8 | 0.2-0.3 | 1.2 |
| 24:0 | 2.2 | 3.5 | -- | 1.1 |
| 24:1+22:5n6 | 1.9 | 3.9 | -- | -- |
| 22:5n3 | 0.3 | 0.1 | 0.2-0.4 | 1.6 |
| 22:6n3 | 8.1 | 8.7 | 0.5-0.9 | 2.2 |

(1) US-4.670.285 and DE-3603000 patents

(2) FR-2553261 patent.

Accordingly, objectives of the present invention are:

– to provide a fat mixture which contains adequate ratios of oleic (18:1n9), linoleic (18:2n6) and alpha-linolenic (18:3n3) fatty acids, as well as adequate levels of long chain PUFA of the n6 and n3 series, and adequate ratios of arachidonic to docosahexaenoic (20:4n6/22:6n3) fatty acids. These ratios are intended to promote adequate nutrition, growth and development of nursing infants, either term or pre-term, and enhance the nutrition, besides complementary prevention and treatment of certain diseases, in adults;

– to provide an edible fat product containing adequate levels of phospholipids, particularly those obtained from the cerebrum of calf or pig, or from other domestic animals;

– to provide formulas for the nutrition of low birth weight newborn infants or at term infants, as well as lactose-free formulas and hydrolyzed protein formulas, with a composition of phospholipids and fatty acids which is similar to that of human milk;

– to provide a variety of diets for adult nutrition, either orally or enterallly, with a specific composition of phospholipids and fatty acids which is similar to that of standard mediterranean diet;

– to provide nutritional products rich in PUFA, both of the n6 and n3 series, independent from the ratio 18:1n9/18:2n6/18:3n3, useful for the dietetic treatment of certain diseases in adults, such as hepatic cirrhosis.

In addition, a fat mixture prepared in accordance with the preceding objectives and characteristics, when it is added with nucleotides and/or nucleosides has been shown to be particularly efective in the treatment of infant diarrheas. The proposed mixture controls the incidence and the duration of the diarrheas.

For example, in experiments conducted with 193 children during a 3 month period, those that received a milk added with a fat mixture according to the invention and nucleotides and/or nucleosides (Milk I) showed the following results when compared to children that received a conventional milk (Milk II):

EP 0 484 266 A2

|                               | Milk I | Milk II |
|-------------------------------|--------|---------|
| Number of children evaluated  | 89     | 84      |
| Episodes of diarrhea          | 38     | 54      |
| Incidence (%)                 | 42.7   | 64.3    |

In the experiment conducted above, also a significant reduction in the duration of the diarrheic episodes was observed:

|                          | Milk I     | Milk II     |
|--------------------------|------------|-------------|
| Average duration (days)  | 6.26       | 8.29        |
| Total days with diarrhea | 219        | 398         |
| Ten days episodes        | 6 (17.1%)  | 13 (27.1%)  |

Therefore, another objective of the present invention is to provide a fat mixture added with a mixture of the nucleosides and/or nucleotides: uridine and/or uridine monophosphate, guanosine and/or guasonine monophosphate, adenosine and/or adenosine monophosphate, cytidine and/or cytidine monophosphate and inosine and/or inosine monophosphate. This embodiment may be used as a specific product or as an additive for another nutritional product, for example milk, which forms part of a diet.

According to the invention, an example of edible fat product is a mixture of phospholipids from cerebrum of domestic animals, preferably calf or pig, and at least one oil of the group of olive, soy, corn, and/or at least an animal fat (milkfat or lard), and/or at least a fish oil, and/or medium chain triglycerides (MCT) obtained from refining of vegetable oils. In this mixture the ratio between oleic/linoleic/alpha-linolenic fatty acids respectively ranges 30-80/3-20/0.3-3, the ratio between arachidonic/docosahexaenoic respectively ranges 0.1-2/0.1-3, and its phospholipids content ranges 23.8-81.5 mg/dl or 2-25 parts by weight per 100 g of mixture.

A fat mixture according to the invention can be administered as a specific product, preferably added with a mixture of nucleotides and/or nucleosides. In addition, a fat mixture according to the invention could be a fraction of an infant formula, or of an adult diet, or generally, of a nutritional product.

The fat mixture and products containing said mixture according to the invention, will be better understood by observation of the following Tables 4 a 19.

Each table discloses non-limitative examples of fat mixtures and infant and adult nutrition products incorporating said mixtures.

Tables 4 to 9 and Examples I to VI, correspond to infant formulas. Tables 10 to 13 and Examples VII to X, correspond to adult diets. Presentation of the respective mixtures in powder or liquid, and containing (b) or not (a) fish oil are distinguishable.

Tables 14 to 19 disclose the composition of the fat mixture, the fatty acid composition in relative and absolute values, and the ratio between 18:1n9/18:2n6/18:3n3 fatty acids and the ratio between 20:4n6/22:6n3 fatty acids.

The fatty acid profiles in the products for infant and adult nutrition are respectively similar to the corresponding essential fatty acids of human milk and mediterranean diet. In addition, the fat mixtures according to the invention have a low content in lauric and myristic acids. The stearic acid content is less than 10% of the relative to the fat and that of the palmitic less than 20%. A higher content of these acids could produce clots, which would inhibit the absorption of fats.

With respect to oleic acid (18:1n9), the energy it provides in the products ranges 15-20% of total energy approximately. The energy supplied by the essential fatty acids linoleic and alpha-linolenic ranges 3-8% and 0.4-0.5%, respectively, of the total energy approximately.

The long chain PUFA content of the mixtures is approximately 0.40% for the arachidonic acid (20:4n6), and about 0.3-0.4% of the total fatty acids for the docosahexaenoic acid (22:6n3). The ratio 20:4n6/22:6n3 in the fat mixtures according to the invention varies in the range 0.1-2/0.1-3, preferably 0.1-1/0.1-1, and the content of 20:5n3 (eicosapentaenoic acid) is not greater than 0.03% in the products for infant nutrition.

The ratios 18:1n9/18:2n6/18:3n3 and 20:4n6/22:6n3 are also similar to those of human milk and mediterranean diet. With regards to the biological source of PUFA used in the fat mixture according to the invention (cerebrum of pig, calf or other domestic animals), the long chain PUFA provided to the diet are associated with phospholipids. Phospholipids represent approximately 70% of the lipids from the cerebrum, the remaining 30% corresponding to cerebrosides, sulfatides and cholesterol (2.2 g %). The fact that the long chain PUFA of the

8

diet are associated to phospholipids constitutes an advantage, especially when taking into account that long chain PUFA in tissues are structural components of membrane phospholipids.

Table 4

Milk formulas for pre-term infants - Example I

|  |  |  | 100 g powder % | 100 ml liquid % |
|---|---|---|---|---|
| Water |  |  | -- | 85 |
| Maltodextrins |  |  | 28.91 | 4.33 |
| Fat mixture |  |  | 26.68 | 3.99 |
|  | a | b |  |  |
| - Olive oil | 43 | 43 |  |  |
| - Milkfat | 23.9 | 23.9 |  |  |
| - MCT | 14.3 | 14.3 |  |  |
| - Soy oil | 14.3 | 14.3 |  |  |
| - Cerebrum phospholipids | 4.5 | 3.2 |  |  |
| - Fish oil | -- | 1.3 |  |  |
| Skim milk (0.05% fat) |  |  | 14.58 | 2.19 |
| Lactalbumin |  |  | 12.13 | 1.82 |
| Lactose |  |  | 11.92 | 1.79 |
| Minerals |  |  | 3.26 | 0.49 |
| Calcium caseinate |  |  | 1.97 | 0.296 |
| Lecithin |  |  | 0.41 | 0.061 |
| Vitamins |  |  | 0.12 | 0.018 |
| Nucleosides and/or nucleotides |  |  | 0.0078 | 0.0012 |
| Ascorbile palmitate |  |  | 0.006 | 0.0009 |
| DL-tocopherol |  |  | 0.001 | 0.0001 |

## Table 5

### Adapted infant milk formulas – Example II

| | | | 100 g powder % | 100 ml liquid % |
|---|---|---|---|---|
| Water | | | -- | 87 |
| Lactose | | | 41.96 | 5.47 |
| Skim milk | | | 18.84 | 2.45 |
| Fat mixture | | | 27.76 | 3.67 |
| | a | b | | |
| - Milkfat | 49.7 | 49.7 | | |
| - Olive oil | 30.5 | 30.5 | | |
| - Soy oil | 10.5 | 10.5 | | |
| - MCT | 4.8 | 4.8 | | |
| - Cerebrum phospholipids | 4.5 | 3.4 | | |
| - Fish oil | -- | 1.1 | | |
| Demineralized whey (65% of proteins) | | | 9.28 | 1.21 |
| Mineral salts | | | 1.11 | 0.14 |
| Lecithin | | | 0.31 | 0.04 |
| Vitamins | | | 0.069 | 0.009 |
| Nucleosides and/or nucleotides | | | 0.0078 | 0.001 |
| DL-tocopherol | | | 0.003 | 0.0004 |
| Ascorbile palmitate | | | 0.001 | 0.0001 |

Table 6

Adapted infant milk continuation formulas - Example III

|  | | | 100 g powder % | 100 ml liquid % |
|---|---|---|---|---|
| Water | | | -- | 85 |
| Skim milk | | | 31.69 | 4.75 |
| Maltodextrins | | | 23.18 | 3.48 |
| Lactose | | | 19.28 | 2.89 |
| Fat mixture | | | 21.03 | 3.15 |
|  | a | b | | |
| - Milkfat | 49.7 | 49.7 | | |
| - Olive oil | 30.5 | 30.5 | | |
| - Soy oil | 10.5 | 10.5 | | |
| - MCT | 4.8 | 4.8 | | |
| - Cerebrum phospholipids | 4.5 | 3.0 | | |
| - Fish oil | -- | 1.5 | | |
| Demineralized whey | | | 4.22 | 0.63 |
| Mineral salts | | | 0.41 | 0.061 |
| Lecithin | | | 0.14 | 0.021 |
| Vitamins | | | 0.069 | 0.01 |
| Nucleosides and/or nucleotides | | | 0.0078 | 0.0012 |
| DL-tocopherol | | | 0.003 | 0.0004 |
| Ascorbile palmitate | | | 0.001 | 0.0001 |

Table 7

Lactose-free adapted infant formulas with milk proteins-
Example IV

| | a | b | 100 g powder % | 100 ml liquid % |
|---|---|---|---|---|
| Water | | | -- | 85 |
| Maltodextrins | | | 58.03 | 8.7 |
| Calcium caseinate (supplemented with L-cistine) | | | 16.7 | 2.51 |
| Fat mixture | | | 22.22 | 3.34 |
| - Milkfat | 49.7 | 49.7 | | |
| - Olive oil | 30.5 | 30.5 | | |
| - Soy oil | 10.5 | 10.5 | | |
| - MCT | 4.8 | 4.8 | | |
| - Cerebrum phospholipids | 4.5 | 2.0 | | |
| - Fish oil | -- | 2.5 | | |
| Mineral salts | | | 2.18 | 0.33 |
| Lecithin | | | 0.69 | 0.103 |
| Vitamins | | | 0.069 | 0.01 |
| Carnitine | | | 0.0089 | 0.0013 |
| Nucleosides and/or nucleotides | | | 0.0078 | 0.0012 |
| DL-tocopherol | | | 0.003 | 0.0004 |
| Ascorbile palmitate | | | 0.001 | 0.0001 |

Table 8

Adapted infant formulas with vegetable proteins - Example V

| | a | b | 100 g powder % | 100 ml liquid % |
|---|---|---|---|---|
| Water | | | -- | 85 |
| Maltodextrins | | | 57.20 | 8.58 |
| Soy protein isolate | | | 16.67 | 2.5 |
| Fat mixture | | | 22.22 | 3.34 |
| - Milkfat | 49.7 | 49.7 | | |
| - Olive oil | 30.5 | 30.5 | | |

| | a | b |
|---|---|---|
| - Soy oil | 10.5 | 10.5 |
| - MCT | 4.8 | 4.8 |
| - Cerebrum phospholipids | 4.5 | 1.5 |
| - Fish oil | -- | 3.0 |

| | 100 g powder | 100 ml liquid |
|---|---|---|
| Mineral salts | 3.04 | 0.46 |
| Lecithin | 0.69 | 0.103 |
| Vitamins | 0.069 | 0.01 |
| Carnitine | 0.0089 | 0.0013 |
| Nucleosides and/or nucleotides | 0.0078 | 0.0012 |
| DL-tocopherol | 0.003 | 0.0004 |
| Ascorbile palmitate | 0.001 | 0.0001 |

**Table 9**

Adapted infant formulas with hypoallergenic protein hydrolyzate
- Example VI

| | 100 g powder % | 100 ml liquid % |
|---|---|---|
| Water | -- | 85 |
| Maltodextrins | 52.48 | 7.87 |
| Fat mixture | 21.27 | 3.19 |

| | a | b |
|---|---|---|
| - Olive oil | 39.1 | 39.1 |
| - MCT | 23.9 | 23.9 |
| - Milkfat | 19.1 | 19.1 |
| - Soy oil | 13.4 | 13.4 |
| - Cerebrum phospholipids | 4.5 | 1.0 |
| - Fish oil | -- | 3.5 |

| | 100 g powder % | 100 ml liquid % |
|---|---|---|
| Lactalbumin enzymatic hydrolyzate | 12.31 | 1.85 |
| Casein enzymatic hydrolyzate | 5.16 | 0.77 |
| Corn starch | 4.87 | 0.73 |
| Minerals | 3.19 | 0.48 |
| Emulsifier | 0.60 | 0.09 |
| Vitamins | 0.069 | 0.01 |
| Lecithin | 0.0231 | 0.0035 |
| Carnitine | 0.0089 | 0.0013 |
| Nucleosides and/or nucleotides | 0.0078 | 0.0012 |
| DL-tocopherol | 0.0038 | 0.0006 |

13

Ascorbile palmitate                  0.0015     0.0002

## Table 10

Normoproteic diet for clinic enteral nutrition of adults-
Example VII

|  | a | b | 100 g powder % | 100 ml liquid % |
|---|---|---|---|---|
| Water |  |  | -- | 78.7 |
| Maltodextrins |  |  | 52.13 | 11.2 |
| Lactalbumin |  |  | 11.63 | 2.48 |
| Fat mixture |  |  | 20.94 | 4.5 |
| - Milkfat | 41.4 | 41.4 |  |  |
| - Olive oil | 33.4 | 33.4 |  |  |
| - Soy oil | 11.5 | 11.5 |  |  |
| - MCT | 9.5 | 9.5 |  |  |
| - Cerebrum phospholipids | 4.5 | 2.4 |  |  |
| - Fish oil | -- | 2.1 |  |  |
| Calcium caseinate |  |  | 10.05 | 2.14 |
| Minerals |  |  | 3.79 | 0.79 |
| Nucleosides and/or nucleotides |  |  | 0.75 | 0.15 |
| Soy lecithin |  |  | 0.66 | -- |
| Emulsifier |  |  | -- | 0.136 |
| Stabilizer |  |  | -- | 0.02 |
| Vitamins |  |  | 0.026 | 0.005 |
| Ascorbile palmitate |  |  | 0.0232 | 0.0008 |
| DL-tocopherol |  |  | 0.0008 | 0.0002 |

## Table 11

Hyperproteic diet for clinic enteral nutrition
Example VIII

|  | 100 g powder % | 100 ml liquid % |
|---|---|---|
| Water | -- | 77.28 |
| Maltodextrins | 50.6 | 11.49 |
| Lactalbumin | 15.96 | 3.64 |

14

| Calcium caseinate | | | 13.08 | 3.14 |
|---|---|---|---|---|
| Fat mixture | | | 15.65 | 1.99 |
| | a | b | | |
| - Milkfat | 41.1 | 41.1 | | |
| - Olive oil | 33.4 | 33.4 | | |
| - Soy oil | 11.5 | 11.5 | | |
| - MCT | 9.5 | 9.5 | | |
| - Cerebrum phospholipids | 4.5 | 2.1 | | |
| - Fish oil | -- | 2.4 | | |
| Minerals | | | 3.41 | 0.68 |
| Nucleosides and/or nucleotides | | | 0.75 | 0.15 |
| Soy lecithin | | | 0.5 | -- |
| Emulsifier | | | -- | 0.11 |
| Stabilizer | | | -- | 0.02 |
| Vitamins | | | 0.026 | 0.005 |
| Ascorbile palmitate | | | 0.0232 | 0.0008 |
| DL-tocopherol | | | 0.0008 | 0.0002 |

Table 12

**MCT peptidic diet for clinical nutrition of adults - Example IX**

| | | | 100 g powder % | 100 ml liquid % |
|---|---|---|---|---|
| Water | | | -- | 77.83 |
| Maltodextrins | | | 51.62 | 11.43 |
| Casein hydrolyzate | | | 25.80 | 5.72 |
| Fat mixture | | | 16.06 | 3.56 |
| | a | b | | |
| - Olive oil | 36.3 | 36.3 | | |
| - MCT | 28.7 | 28.7 | | |
| - Milkfat | 18.1 | 18.1 | | |
| - Soy oil | 12.4 | 12.4 | | |
| - Cerebrum phospholipids | 4.5 | 1.9 | | |
| - Fish oil | -- | 2.6 | | |
| Minerals | | | 5.02 | 1.11 |
| Nucleosides and/or nucleotides | | | 0.75 | 0.17 |
| Soy lecithin | | | 0.50 | -- |
| Emulsifier | | | -- | 0.11 |

| | 100 g powder % | 100 ml liquid % |
|---|---|---|
| L-cistine | 0.20 | 0.04 |
| Stabilizer | -- | 0.02 |
| Vitamins | 0.026 | 0.0058 |
| Ascorbile palmitate | 0.0232 | 0.0051 |
| DL-tocopherol | 0.0008 | 0.0002 |

**Table 13**

Dietetic treatment of hepatic diseases in clinical nutrition- Example X

| | a | b | 100 g powder % | 100 ml liquid % |
|---|---|---|---|---|
| Water | | | -- | 76.36 |
| Maltodextrins | | | 72.13 | 17.04 |
| Fat mixture | | | 7.48 | 1.77 |
| - MCT | 59.2 | 59.2 | | |
| - Corn oil | 36.3 | 36.3 | | |
| - Cerebrum phospholipids | 4.5 | 2.6 | | |
| - Fish oil | -- | 1.9 | | |
| Lactalbumin | | | 7.26 | 1.72 |
| Calcium caseinate | | | 6.27 | 1.48 |
| Minerals | | | 2.94 | 0.69 |
| L-leucine | | | 1.16 | 0.27 |
| L-valine | | | 0.87 | 0.21 |
| L-isoleucine | | | 0.87 | 0.21 |
| Nucleosides and/or nucleotides | | | 0.75 | 0.18 |
| Soy lecithin | | | 0.22 | -- |
| Emulsifier | | | -- | 0.05 |
| Stabilizer | | | -- | 0.01 |
| Vitamins | | | 0.026 | 0.006 |
| Ascorbile palmitate | | | 0.0197 | 0.005 |
| DL-tocopherol | | | 0.0003 | 0.00007 |

### Table 14

Products for infant nutrition - Composition of fatty material

| Examples | I | | II | | III | |
|---|---|---|---|---|---|---|
| - g of fat/100 g of product | 28 | | 29 | | 21.3 | |
| - % of fat in mixture | a | b | a | b | a | b |
| Milkfat | 23.9 | 23.9 | 49.7 | 49.7 | 49.7 | 49.7 |
| Olive oil | 43 | 43 | 30.5 | 30.5 | 30.5 | 30.5 |
| Soy oil | 14.3 | 14.3 | 10.5 | 10.5 | 10.5 | 10.5 |
| MCT | 14.3 | 14.3 | 4.8 | 4.8 | 4.8 | 4.8 |
| Cerebrum phospholipids | 4.5 | 3.2 | 4.5 | 3.4 | 4.5 | 3.0 |
| Fish oil | -- | 1.3 | -- | 1.1 | -- | 1.5 |

### Table 14 bis

Products for infant nutrition - Composition of fatty material

| Examples | IV | | V | | VI | |
|---|---|---|---|---|---|---|
| - g of fat/100 g of product | 22.9 | | 23 | | 22 | |
| - % fat in mixture | a | b | a | b | a | b |
| Milkfat | 49.7 | 49.7 | 49.7 | 49.7 | 19.1 | 19.1 |
| Olive oil | 30.5 | 30.5 | 30.5 | 30.5 | 39.1 | 39.1 |
| Soy oil | 10.5 | 10.5 | 10.5 | 10.5 | 13.4 | 13.4 |
| MCT | 4.8 | 4.8 | 4.8 | 4.8 | 23.9 | 23.9 |
| Cerebrum phospholipids | 4.5 | 2.0 | 4.5 | 1.5 | 4.5 | 1.0 |
| Fish oil | -- | 2.5 | -- | 3.0 | -- | 3.5 |

### Table 15

Adult nutrition products - Composition of fatty material

| Examples | VII | | VIII | | IX | | X | |
|---|---|---|---|---|---|---|---|---|
| - g of fat/100 g of product | 22 | | 16.6 | | 16.6 | | 7.7 | |
| - % fat in mixture | a | b | a | b | a | b | a | b |
| Milkfat | 41.4 | 41.4 | 41.1 | 41.1 | 18.1 | 18.1 | - | - |
| Olive oil | 33.4 | 33.4 | 33.4 | 33.4 | 36.3 | 36.3 | - | - |
| Soy oil | 11.5 | 11.5 | 11.5 | 11.5 | 12.4 | 12.4 | - | - |
| MCT | 9.5 | 9.5 | 9.5 | 9.5 | 28.7 | 28.7 | 59.2 | 59.2 |
| Corn oil | - | - | - | - | - | - | 36.3 | 36.3 |

17

```
Cerebrum phospholipids 4.5  2.4    4.5  2.1    4.5  1.9    4.5  2.6
Fish oil                -   2.1     -   2.4     -   2.6     -   1.9
```

## Table 16
**Relative fatty acid composition of infant nutrition products**

| Examples | I | | II | | III | |
|---|---|---|---|---|---|---|
| | <u>a</u> | <u>b</u> | <u>a</u> | <u>b</u> | <u>a</u> | <u>b</u> |
| 8:0 | 7.20 | 7.20 | 2.40 | 2.39 | 2.35 | 2.35 |
| 10:0 | 8.90 | 8.90 | 3.80 | 3.72 | 3.70 | 3.70 |
| 12:0 | 1.20 | 1.20 | 1.90 | 1.91 | 1.80 | 1.80 |
| 14:0 | 3.10 | 3.07 | 5.20 | 2.11 | 5.00 | 4.06 |
| 16:0 | 15.05 | 15.45 | 18.80 | 18.67 | 19.00 | 19.40 |
| 16:1n7 | 1.60 | 1.67 | 1.70 | 1.75 | 1.60 | 1.68 |
| 18:0 | 5.40 | 5.08 | 8.03 | 7.58 | 7.90 | 7.53 |
| 18:1n9 | 39.20 | 39.14 | 39.70 | 39.05 | 39.80 | 39.73 |
| 18:2n6 | 14.50 | 14.52 | 13.80 | 13.27 | 13.85 | 13.87 |
| 18:3n3 | 1.30 | 1.30 | 1.10 | 1.10 | 1.10 | 1.10 |
| 20:1+18:4n3 | 0.07 | 0.10 | 0.07 | 0.09 | 0.07 | 0.10 |
| 20:2n6 | 0.03 | 0.02 | 0.03 | 0.02 | 0.03 | 0.04 |
| 20:3n6 | 0.03 | 0.03 | 0.03 | 0.03 | 0.03 | 0.03 |
| 20:4n6 | 0.40 | 0.29 | 0.40 | 0.30 | 0.40 | 0.27 |
| 20:5n3 | 0.03 | 0.14 | 0.03 | 0.12 | 0.03 | 0.16 |
| 22:4n6 | 0.23 | 0.16 | 0.23 | 0.17 | 0.23 | 0.15 |
| 24:0 | 0.10 | 0.08 | 0.10 | 0.08 | 0.10 | 0.08 |
| 24:1+22:5n6 | 0.10 | 0.08 | 0.10 | 0.08 | 0.10 | 0.08 |
| 22:5n3 | 0.02 | 0.03 | 0.02 | 0.03 | 0.02 | 0.03 |
| 22:6n3 | 0.36 | 0.42 | 0.36 | 0.41 | 0.36 | 0.43 |

Ratio 18:1n9/18:2n9/18:3n3
- Example  I   a) 30/11.1/1    b) 30.1/11.2/1.0
- Example  II  a) 36/12.5/1    b) 35.5/12.0/1.0
- Example  III a) 36/12.5/1    b) 36.1/12.6/.01

Ratio 20:4n6/22:6n3
- Example  I   a) 1.11/1    b) 0.69/1
- Example  II  a) 1.11/1    b) 0.73/1
- Example  III a) 1.11/1    b) 0.63/1

## Table 16 bis

Relative fatty acid composition of infant nutrition products

| Examples | IV | | V | | VI | |
|---|---|---|---|---|---|---|
| | a | b | a | b | a | b |
| 8:0 | 2.50 | 2.38 | 2.40 | 2.38 | 9.50 | 9.45 |
| 10:0 | 3.60 | 3.72 | 3.70 | 3.72 | 14.00 | 13.94 |
| 12:0 | 1.90 | 1.91 | 1.90 | 2.00 | 1.30 | 1.33 |
| 14:0 | 5.10 | 5.18 | 5.10 | 5.18 | 2.10 | 2.13 |
| 16:0 | 18.90 | 18.73 | 19.00 | 18.74 | 12.50 | 12.66 |
| 16:1n7 | 1.70 | 1.81 | 1.70 | 1.36 | 1.30 | 1.38 |
| 18:0 | 8.15 | 7.42 | 8.10 | 7.37 | 4.40 | 3.97 |
| 18:1n9 | 39.60 | 39.16 | 39.70 | 39.13 | 35.80 | 35.56 |
| 18:2n6 | 13.82 | 13.35 | 13.75 | 13.38 | 14.30 | 14.46 |
| 18:3n3 | 1.10 | 1.05 | 1.10 | 1.11 | 1.30 | 1.31 |
| 20:1+18:4n3 | 0.07 | 0.13 | 0.07 | 0.14 | 0.07 | 0.14 |
| 20:2n6 | 0.03 | 0.01 | 0.03 | 0.01 | 0.03 | 0.01 |
| 20:3n6 | 0.03 | 0.03 | 0.03 | 0.03 | 0.03 | 0.03 |
| 20:4n6 | 0.40 | 0.18 | 0.40 | 0.13 | 0.40 | 0.09 |
| 20:5n3 | 0.03 | 0.25 | 0.03 | 0.29 | 0.03 | 0.34 |
| 22:4n6 | 0.23 | 0.10 | 0.23 | 0.08 | 0.23 | 0.05 |
| 24:0 | 0.10 | 0.04 | 0.10 | 0.03 | 0.10 | 0.02 |
| 24:1+22:5n6 | 0.10 | 0.04 | 0.10 | 0.03 | 0.10 | 0.02 |
| 22:5n3 | 0.02 | 0.04 | 0.02 | 0.04 | 0.02 | 0.05 |
| 22:6n3 | 0.36 | 0.47 | 0.36 | 0.49 | 0.36 | 0.51 |

Ratio 18:1n9/18:2n6/18:3n3

- Example  IV  a) 36/12.5/1       b) 37.3/12.7/1.0
- Example  V   a) 36/12.5/1       b) 35.2/12.0/1.0
- Example  VI  a) 27.5/11/1       b) 27.1/11.0/1.0

Ratio 20:4n6/22:6n3

- Example IV   a) 1.1/1   b) 0.38/1
- Example V    a) 1.1/1   b) 0.26/1
- Example VI   a) 1.1/1   b) 0.18/1

Table 17

Relative fatty acid composition of adult nutrition products

| Examples | VII | | VIII | | IX | | X | |
|---|---|---|---|---|---|---|---|---|
| | a | b | a | b | a | b | a | b |
| 6:0 | 0.10 | 0.10 | 0.10 | 0.10 | 0.3 | 0.10 | 0.60 | 0.60 |
| 8:0 | 4.20 | 4.20 | 4.20 | 4.10 | 11.25 | 10.94 | 22.30 | 21.29 |
| 10:0 | 6.30 | 6.30 | 6.20 | 6.20 | 16.50 | 16.30 | 32.96 | 31.47 |
| 12:0 | 1.70 | 1.70 | 1.60 | 1.60 | 1.50 | 1.40 | 1.75 | 1.70 |
| 14:0 | 4.70 | 4.56 | 4.70 | 4.70 | 2.20 | 2.10 | 0.18 | 0.18 |
| 16:0 | 17.60 | 17.86 | 17.70 | 17.68 | 11.90 | 11.91 | 4.75 | 4.75 |
| 16:1n7 | 1.60 | 1.72 | 1.60 | 1.79 | 1.15 | 1.36 | 0.10 | 0.26 |
| 18:0 | 6.90 | 6.65 | 7.10 | 6.61 | 4.10 | 3.80 | 2.15 | 2.18 |
| 18:1n9 | 39.20 | 38.92 | 39.40 | 39.10 | 33.30 | 33.00 | 10.15 | 10.25 |
| 18:2n6 | 12.64 | 12.11 | 12.75 | 12.11 | 12.70 | 12.64 | 18.06 | 17.22 |
| 18:3n3 | 1.20 | 1.21 | 1.10 | 1.21 | 1.20 | 1.22 | 3.20 | 3.21 |
| 20:1 + 18:4n3 | 0.07 | 0.10 | 0.07 | 0.10 | 0.07 | 0.03 | 0.07 | 0.03 |
| 20:2n6 | 0.03 | 0.01 | 0.03 | 0.01 | 0.03 | 0.01 | 0.03 | 0.01 |
| 20:3n6 | 0.03 | 0.01 | 0.03 | 0.01 | 0.03 | 0.01 | 0.03 | 0.01 |
| 20:4n6 | 0.40 | 0.24 | 0.40 | 0.22 | 0.40 | 0.20 | 0.40 | 0.25 |
| 20:5n3 | 0.03 | 0.22 | 0.03 | 0.25 | 0.03 | 0.27 | 0.03 | 0.20 |
| 22:4n6 | 0.23 | 0.16 | 0.23 | 0.15 | 0.23 | 0.14 | 0.23 | 0.17 |
| 24:0 | 0.10 | 0.03 | 0.10 | 0.03 | 0.10 | 0.02 | 0.10 | 0.03 |
| 24:1 + 22:5n6 | 0.10 | 0.03 | 0.10 | 0.03 | 0.10 | 0.02 | 0.10 | 0.03 |
| 22:5n3 | 0.02 | 0.02 | 0.02 | 0.05 | 0.02 | 0.06 | 0.02 | 0.04 |
| 22:6n3 | 0.36 | 0.45 | 0.36 | 0.46 | 0.36 | 0.47 | 0.36 | 0.48 |

Ratio 18:1n9/18:2n6/18:3n3

- Example VII    a) 32.7/10.6/1    b) 32.2/10/1
- Example VIII   a) 35.8/11.6/1    b) 32.3/10/1
- Example IX     a) 27.8/10.6/1    b) 27.0/10.4/1
- Example X      a) 3.2/5.6/1      b) 3.2/5.4/1

Ratio 20:4n6/22:6n3

- Example VII    a) 1.1/1    b) 0.53/1
- Example VIII   a) 1.1/1    b) 0.48/1
- Example IX     a) 1.1/1    b) 0.42/1
- Example X      a) 1.1/1    b) 0.52/1

Table 18

Fatty acid composition of infant nutrition products (4.5% cerebrum phospholipids + 95.5% primitive fat) in g/100 g of product

| Examples | I a | I b | II a | II b | III a | III b |
|---|---|---|---|---|---|---|
| 8:0 | 1.714 | 1.714 | 0.592 | 0.589 | 0.434 | 0.434 |
| 10:0 | 2.118 | 2.118 | 0.937 | 0.918 | 0.688 | 0.688 |
| 12:0 | 0.286 | 0.286 | 0.468 | 0.471 | 0.344 | 0.344 |
| 14:0 | 0.740 | 0.731 | 1.282 | 0.520 | 0.941 | 0.764 |
| 16:0 | 3.570 | 3.68 | 4.634 | 4.605 | 3.404 | 3.476 |
| 16:1n7 | 0.381 | 0.397 | 0.419 | 0.432 | 0.308 | 0.323 |
| 17:0 | -- | -- | -- | -- | -- | -- |
| 18:0 | 1.285 | 1.210 | 1.972 | 1.870 | 1.448 | 1.380 |
| 18:1n9 | 9.330 | 9.317 | 9.786 | 9.632 | 7.189 | 7.175 |
| 18:2n6 | 3.451 | 3.457 | 3.402 | 3.273 | 2.498 | 2.502 |
| 18:3n3 | 0.309 | 0.309 | 0.271 | 0.271 | 0.199 | 0.199 |
| 20:1+18:4n3 | 0.017 | 0.024 | 0.017 | 0.022 | 0.013 | 0.018 |
| 20:2n6 | 0.007 | 0.005 | 0.007 | 0.005 | 0.005 | 0.006 |
| 20:3n6 | 0.007 | 0.007 | 0.007 | 0.007 | 0.005 | 0.005 |
| 20:4n6 | 0.095 | 0.069 | 0.099 | 0.074 | 0.072 | 0.049 |
| 20:5n3 | 0.007 | 0.033 | 0.007 | 0.029 | 0.005 | 0.026 |
| 22:4n6 | 0.055 | 0.038 | 0.057 | 0.042 | 0.041 | 0.027 |
| 24:0 | 0.024 | 0.019 | 0.025 | 0.020 | 0.018 | 0.014 |
| 24:1+22:5n6 | 0.024 | 0.019 | 0.025 | 0.020 | 0.018 | 0.014 |
| 22:5n3 | 0.005 | 0.007 | 0.005 | 0.007 | 0.004 | 0.006 |
| 22:6n3 | 0.086 | 0.1000 | 0.089 | 0.101 | 0.065 | 0.078 |

Table 18 bis

Fatty acid composition of infant nutrition products (4.5% cerebrum phospholipids + 95.5% primitive fat) in g/100 g of product

| Examples | IV a | IV b | V a | V b | VI a | VI b |
|---|---|---|---|---|---|---|
| 8:0 | 0.469 | 0.446 | 0.469 | 0.467 | 0.776 | 1.767 |
| 10:0 | 0.743 | 0.698 | 0.743 | 0.727 | 2.618 | 2.606 |
| 12:0 | 0.371 | 0.358 | 0.371 | 0.391 | 0.243 | 0.249 |
| 14:0 | 1.017 | 0.971 | 1.017 | 1.012 | 0.393 | 0.398 |
| 16:0 | 3.675 | 3.514 | 3.675 | 3.662 | 2.337 | 2.367 |
| 16:1n7 | 0.332 | 0.340 | 0.332 | 0.265 | 0.243 | 0.258 |

| | | | | | | |
|---|---|---|---|---|---|---|
| 17:0 | -- | -- | -- | -- | -- | -- |
| 18:0 | 1.564 | 1.391 | 1.564 | 1.440 | 0.823 | 0.742 |
| 18:1n9 | 7.761 | 7.346 | 7.761 | 7.647 | 6.695 | 6.648 |
| 18:2n6 | 2.698 | 2.504 | 2.698 | 2.615 | 2.674 | 2.703 |
| 18:3n3 | 0.215 | 0.197 | 0.215 | 0.217 | 0.243 | 0.245 |
| 20:1+18:4n3 | 0.014 | 0.024 | 0.014 | 0.027 | 0.013 | 0.026 |
| 20:2n6 | 0.006 | 0.002 | 0.006 | 0.002 | 0.006 | 0.002 |
| 20:3n6 | 0.006 | 0.006 | 0.006 | 0.006 | 0.006 | 0.006 |
| 20:4n6 | 0.078 | 0.034 | 0.078 | 0.025 | 0.080 | 0.017 |
| 20:5n3 | 0.006 | 0.047 | 0.006 | 0.057 | 0.006 | 0.063 |
| 22:4n6 | 0.045 | 0.019 | 0.045 | 0.016 | 0.043 | 0.009 |
| 24:0 | 0.019 | 0.007 | 0.019 | 0.006 | 0.019 | 0.004 |
| 24:1+22:5n6 | 0.019 | 0.007 | 0.019 | 0.006 | 0.019 | 0.004 |
| 22:5n3 | 0.003 | 0.007 | 0.003 | 0.008 | 0.004 | 0.009 |
| 22:6n3 | 0.070 | 0.088 | 0.070 | 0.096 | 0.067 | 0.095 |

Table 19

Fatty acid composition of clinic enteral nutrition products for adults, in g/100 g of product

| Examples | VII | | VIII | | IX | | X | |
|---|---|---|---|---|---|---|---|---|
| | a | b | a | b | a | b | a | b |
| 6:0 | 0.019 | 0.019 | 0.014 | 0.014 | 0.042 | 0.014 | 0.39 | 0.039 |
| 8:0 | 0.785 | 0.785 | 0.593 | 0.593 | 1.587 | 1.543 | 1.459 | 1.393 |
| 10:0 | 1.178 | 1.178 | 0.889 | 0.889 | 2.328 | 2.300 | 2.153 | 2.059 |
| 12:0 | 0.318 | 0.318 | 0.240 | 0.240 | 0.212 | 0.197 | 0.114 | 0.046 |
| 14:0 | 0.879 | 0.853 | 0.663 | 0.659 | 0.310 | 0.296 | 0.012 | 0.012 |
| 16:0 | 3.291 | 3.33 | 2.483 | 2.481 | 1.679 | 1.680 | 0.311 | 0.311 |
| 16:1n7 | 0.299 | 0.322 | 0.226 | 0.251 | 0.162 | 0.192 | 0.006 | 0.017 |
| 18:0 | 1.290 | 1.243 | 0.974 | 0.987 | 0.578 | 0.536 | 0.141 | 0.142 |
| 18:1n9 | 7.330 | 7.277 | 5.531 | 5.489 | 4.699 | 4.655 | 0.664 | 0.629 |
| 18:2n6 | 2.364 | 2.265 | 1.783 | 1.700 | 1.791 | 1.783 | 1.182 | 1.057 |
| 18:3n3 | 0.224 | 0.226 | 0.169 | 0.169 | 0.169 | 0.171 | 0.209 | 0.197 |
| 20:1 + 18:4n3 | 0.013 | 0.019 | 0.010 | 0.014 | 0.010 | 0.003 | 0.005 | 0.002 |
| 20:2n6 | 0.006 | 0.001 | 0.004 | 0.001 | 0.004 | 0.001 | 0.002 | 0.001 |
| 20:3n6 | 0.006 | 0.001 | 0.004 | 0.001 | 0.004 | 0.001 | 0.002 | 0.001 |
| 20:4n6 | 0.075 | 0.037 | 0.056 | 0.027 | 0.056 | 0.025 | 0.026 | 0.002 |
| 20:5n3 | 0.006 | 0.041 | 0.004 | 0.030 | 0.004 | 0.033 | 0.002 | 0.012 |

```
22:4n6   0.043   0.030   0.032   0.018   0.032   0.017   0.015   0.010
24:0     0.019   0.006   0.014   0.004   0.014   0.003   0.006   0.002
24:1 +
22:5n6   0.019   0.006   0.014   0.004   0.014   0.003   0.006   0.002
22:5n3   0.004   0.009   0.003   0.006   0.003   0.007   0.001   0.002
22:6n3   0.067   0.084   0.050   0.057   0.050   0.058   0.023   0.029
```

## Claims

1.- Fat mixture for infant and adult nutrition, characterised by a ratio between oleic/linoleic/alpha-linolenic fatty acids in the respective ranges of 30-80/3-20/0.3-3.

2.- Fat mixture for infant and adult nutrition, characterised by a ratio between arachidonic/docosahexaenoic fatty acids in the respective ranges of 0.1-2/0.1-3.

3.- Fat mixture for infant and adult nutrition, characterised by a phospholipids content in the range of 23.8-81.5 mg/dl.

4.- Fat mixture for infant and adult nutrition, characterised by a phospholipids content in the range of 2-25 parts by weight per 100 g of mixture.

5.- Fat mixture for infant and adult nutrition, according to claims 1 to 4, characterised in that its phospholipids content derives mainly from cerebrum phospholipids of mammals.

6.- Fat mixture for infant and adult nutrition, according to the preceding claims, characterised in that it comprises a mixture of phospholipids from cerebrum of mammals, at least one oil from the group of olive, soy and corn, and/or at least an animal fat, and/or at least a fish oil, and/or medium chain triglycerides, in which the ratio between oleic/linoleic/alpha-linolenic fatty acids is in the respective ranges of 30-80/3-20/0.3-3, the ratio between arachidonic/docosahexaenoic fatty acids is in the respective ranges of 0.1-2/0.1-3 and its phospholipids content in the range of 23.8-81.5 mg/dl.

7.- Fat mixture for infant and adult nutrition, according to the preceding claims, characterised in that it comprises a mixture of phospholipids from cerebrum of mammals, at least one oil from the group of olive, soy and corn, and/or at least an animal fat, and/or at least a fish oil, and/or medium chain triglycerides, in which the ratio between oleic/linoleic/alpha-linolenic fatty acids is in the respective ranges of 30-80/3-20/0.3-3, the ratio between arachidonic/docosahexaenoic fatty acids is in the respective ranges of 0.1-2/0.1-3 and its phospholipids content in the range of 2-25 parts by weight per 100 g of mixture.

8.- A nutritional product for infants and adults, characterised by comprising a fat mixture according to anyone of the preceding claims.

9.- A fat mixture according to anyone of the preceding claims characterised by being added with a mixture of the nucleosides and/or nucleotides: uridine and/or uridine monophosphate, guanosine and/or guasonine monophosphate, adenosine and/or adenosine monophosphate, cytidine and/or cytidine monophosphate and inosine and/or inosine monophosphate.

10.- A nutritional product for infants and adults, characterised by comprising a fat mixture according to anyone of the preceding claims and a mixture of the nucleosides and/or nucleotides: uridine and/or uridine monophosphate, guanosine and/or guasonine monophosphate, adenosine and/or adenosine monophosphate, cytidine and/or cytidine monophosphate and inosine and/or inosine monophosphate.

11.- A fat mixture according to anyone of the preceding claims, characterised by its use on the dietetic treatment of hepatic cirrhosis.

12.- A nutritional product comprising a fat mixture according to anyone of the preceding claims, characterised by its use on the dietetic treatment of hepatic cirrhosis.

13.- A mixture of fat and of nucleotides and/or nucleosides, according to claim 9, characterised by its use on the dietetic treatment of diarrheas.

14.- A nutritional product comprising a mixture of fat and of nucleotides and/or nucleosides, according to claim 10, characterised by its use on the dietetic treatment of diarrheas.